# FASCICULE DE BREVET EUROPEEN

(11) **EP 1 046 376 B1**
(45) Date de publication et mention de la délivrance du brevet: **10.11.2004**
(21) Numéro de dépôt: 00420076.2
(22) Date de dépôt: 18.04.2000
(51) Int. Cl.: A61B 17/86

(54) **Vis d'ostéosynthèse à compression et ancillaire de mise en oeuvre**
Kompressionsknochenschraube und Hilfinstrumente zu ihrer Befestigung
Compression screw for Osteosynthesis and ancillary equipment for fitting same

(30) Priorité: 22.04.1999 FR 9905399
(43) Date de publication de la demande: 25.10.2000
(73) Titulaire: Newdeal S.A., 69006 Lyon (FR)
(72) Inventeur: Giet, Jean-Christophe Alain, 69008 Lyon (FR); Knevels, Theo Jan Maria, 1850 Grimbergen (BE); Fourcault, Eric Stéphane, 69005 Lyon (FR)
(74) Mandataire: Martin, Didier

(56) Documents cités:
- WO-A-91/09572
- FR-A- 2 699 065
- FR-A- 2 728 778
- FR-A- 2 745 709
- JP-A- 10 052 439

## Description

La présente invention se rapporte au domaine technique de la chirurgie osseuse, et en particulier aux appareils d'ostéosynthèse destinés à assurer le rapprochement, la mise sous compression puis la réunion de fragments d'os fracturés au moyen de vis, boulons, clous, plaques métalliques ou autres.

La présente invention concerne une vis d'ostéosynthèse à compression destinée à la mise sous compression de fragments d'os fracturés, ladite vis comprenant d'une part un corps de vis présentant un filetage avec une zone distale et une zone proximale, et d'autre part une tête de vis destinée à être enfilée sur ledit corps de vis et à coopérer avec la zone proximale par un taraudage interne congruent avec le filetage dudit corps de vis, ladite tête de vis comportant également un filetage externe de pas inférieur à celui du corps de vis.

La présente invention concerne également un ancillaire pour la mise en oeuvre d'une vis d'ostéosynthèse à compression.

Il est ainsi déjà connu d'assurer la réunion de fragments d'os à l'aide d'une vis dont le corps est percé sur toute sa longueur et qui comprend un filetage externe sur la totalité de sa longueur. La vis est associée à une tête de vis distincte qui présente un puits interne traversant de manière à pouvoir être enfilée sur le corps de vis. Le puits interne est pourvu d'un taraudage congruent avec le filetage du corps de vis de manière à pouvoir coopérer avec ce dernier et se déplacer sur toute la longueur du filetage pour venir en position de butée dans la zone proximale du corps de vis. La tête de vis comporte également un filetage externe de pas inférieur au filetage du corps de vis.

Un tel dispositif donne de manière générale des résultats satisfaisants en matière de traitement de l'ostéosynthèse puisqu'il autorise, par vissage du corps de vis à travers les fragments osseux, un rapprochement relatif des fragments osseux permettant de réunir, par jointoiement, au niveau du trait de fracture, les divers fragments osseux. Ce dispositif permet également une légère mise en compression des fragments osseux en raison de l'existence d'un pas de vis externe différentiel sur la tête de vis relativement au filetage du corps de vis. En effet, l'existence d'un pas de vis différentiel au niveau du filet extérieur de la tête de vis permet, lorsque cette dernière commence à pénétrer dans la partie osseuse, un rapprochement relatif entre les fragments osseux impliquant une mise en compression. Une telle compression peut être légèrement augmentée par la mise en rotation seule de la tête de vis qui permet de contrôler ou d'augmenter sensiblement la compression.

Un tel dispositif souffre néanmoins d'inconvénients limitant la compression des fragments d'os en raison de l'existence du filetage du corps de vis qui s'oppose à la mise en compression en raison de son ancrage direct dans les fragments d'os. En outre, la présence du filetage du corps de vis peut être à l'origine de traumatismes osseux néfastes à la bonne résorption de la fracture.

On connaît également dans l'art antérieur d'autres types de vis d'ostéosynthèse à compression à pas double, de telles vis ne comportant pas de tête de vis indépendante et ne permettant en conséquence qu'une compression modérée des fragments d'os à réunir.

Le document FR-2 728 778 décrit une vis correspondant au préambule de la revendication principale.

Le document FR-2 745 709 décrit une vis d'ostéosynthèse comportant un corps de vis présentant trois secteurs, à savoir une zone distale filetée, une zone proximale filetée, ainsi qu'une zone intermédiaire lisse située entre les zones proximale et distale. La vis décrite par ce document comprend également une tête de vis destinée à être enfilée sur le corps de vis par la zone proximale.

La présente invention se propose en conséquence de porter remède aux différents inconvénients énumérés précédemment et de fournir une nouvelle vis d'ostéosynthèse à compression dont la mise en place est facilitée et les capacités de compression des fragments osseux à réunir augmentées.

Un autre objet de l'invention est de fournir une nouvelle vis d'ostéosynthèse à compression dont la fabrication est simplifiée, le coût réduit, et les risques de traumatismes osseux maîtrisés.

Un autre objet de l'invention vise à proposer une nouvelle vis d'ostéosynthèse à compression présentant une bonne tenue mécanique tout en réduisant les risques d'apparition de phénomènes de résorptions.

Un autre objet de l'invention vise à proposer un nouvel ancillaire pour la mise en oeuvre d'une vis d'ostéosynthèse à compression permettant en cours d'opération d'éviter de manière fiable et en toute sécurité, la sortie de la tête de vis hors de son filetage de vissage sur le corps de vis.

Les objets assignés à l'invention sont atteints à l'aide d'une vis d'ostéosynthèse conforme au libellé de la revendication 1. Les objets assignés à l'invention sont atteints à l'aide d'un ancillaire pour la mise en oeuvre de la vis d'ostéosynthèse selon l'invention comportant un premier tournevis destiné à assurer le vissage du corps de vis, et un second tournevis destiné à assurer le vissage de la tête de vis, le premier tournevis étant emmanché axialement dans le second tournevis pour permettre le vissage simultané du corps de vis et de la tête de vis et séparable de ce dernier pour permettre le vissage individuel de la tête de vis, caractérisé en ce que le second tournevis est pourvu à sa partie terminale travaillante d'un lamage interne destiné à recevoir la collerette du corps de vis en position de butée contre son fond, ledit lamage étant d'une profondeur telle que lorsque la collerette vient en butée contre le fond lors du vissage de la tête de vis, le vissage de cette dernière est automatiquement bloqué avant d'avoir atteint le dernier filet du filetage de manière à empêcher la sortie de la tête de vis hors dudit filetage.

Les objets assignés à l'invention seront explicités plus en détail à la lecture de la description qui suit à l'aide des dessins annexés ci-après, donnés à titre purement illustratif et non limitatif, dans lesquels :
- la figure 1 représente, selon une vue en perspective éclatée, une vis d'ostéosynthèse à compression conforme à l'invention avec un corps de vis et une tête de vis séparés.
- la figure 2 montre, selon une vue de face, une vis d'ostéosynthèse à compression conforme à l'invention montrant le montage de la tête de vis en position de butée sur le corps de vis.
- Les figures 3 et 4 montrent, selon des vues partielles schématiques, les conformations géométriques préférentielles, respectivement des filetages de la partie distale et proximale d'une vis d'ostéosynthèse à compression conforme à l'invention.
- La figure 5 montre, selon une vue en perspective générale, un ancillaire conforme à l'invention, les deux instruments le composant étant en position séparée l'un de l'autre.
- La figure 6 montre, selon une vue en perspective partielle, un détail de réalisation de l'extrémité de travail d'un ancillaire conforme à l'invention lorsque les deux éléments le composant sont emmanchés l'un dans l'autre.
- La figure 7 illustre, selon une vue en coupe transversale longitudinale partielle effectuée dans l'élément ancillaire responsable du vissage de la tête de vis, la position de butée dudit élément.

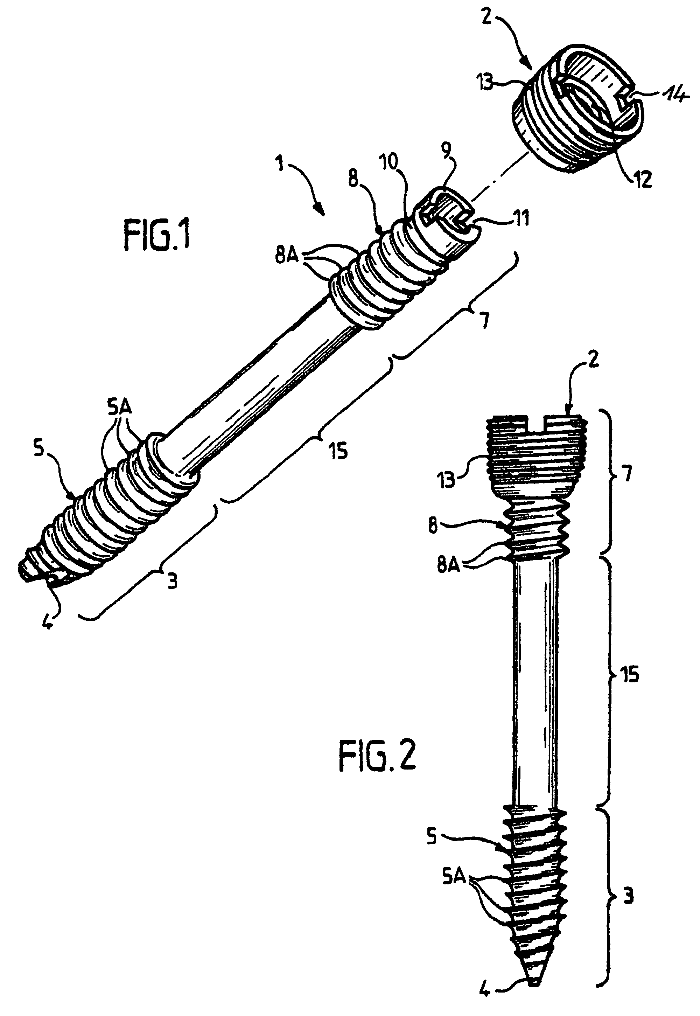

Les figures 1 et 2 montrent des vues générales d'une vis d'ostéosynthèse à compression conforme à l'invention. Une telle vis est composée de deux éléments distincts destinés à être assemblés et comprenant un corps de vis 1 et une tête de vis 2. Ces deux éléments sont réalisés en matériaux métalliques traités spécialement choisis pour leur utilisation dans le domaine chirurgical.

Le corps de vis 1 se présente sous la forme générale d'un corps sensiblement cylindrique de révolution, de préférence percé sur toute sa longueur. Le corps de vis 1 comprend une zone distale 3 dont l'extrémité est pourvue d'un biseau 4 pour faciliter la pénétration dans la partie osseuse et dont la partie externe est pourvue d'un filetage 5 dit osseux dont les caractéristiques géométriques peuvent varier mais sont choisies pour faciliter une bonne pénétration dans les fragments osseux ainsi qu'une bonne tenue mécanique.

Le corps de vis 1 comporte également une zone proximale 7 de même diamètre externe que la zone distale 3 et comportant un filetage externe 8 dont le pas est choisi de manière à permettre le montage et le déplacement de la tête de vis 2. La zone proximale 7 est pourvue à son extrémité d'une collerette 9 de diamètre sensiblement supérieur à la partie supérieure 10 de la zone proximale 7 dépourvue de filets de manière à former une butée. La collerette 9 est pourvue de deux encoches 11 diamétralement opposées ménagées dans sa masse de manière à permettre l'introduction de la partie travaillante d'un outil de mise en place du corps de vis 1, tel qu'un tournevis.

Selon l'invention, le pas du filetage 5 est identique au pas du filetage 8, lesdits filetages étant par ailleurs constants.

La tête de vis 2 conforme à l'invention est destinée à être enfilée sur le corps de vis 1 par la zone distale 3 et à coopérer avec la zone proximale 7. A cet effet, la tête de vis 2 se présente sous la forme d'un corps de révolution sensiblement cylindrique, et de préférence légèrement tronconique en considérant ces génératrices externes, ladite tête de vis 2 présentant un puits traversant interne pourvu d'un taraudage 12. Ce dernier est congruent avec le filetage 8 de la zone proximale 7 de manière à coopérer par vissage avec ce dernier. Le taraudage 12 comporte également un pas identique à celui du filetage 5 de la zone distale 3 afin de permettre à la tête de vis 2 d'être enfilée et déplacée par vissage sur ce dernier. La tête de vis est en outre pourvue de deux encoches 14 diamétralement opposées qui sont ménagées dans la masse et qui débouchent en partie supérieure de la tête.

Un tel montage permet en conséquence d'enfiler la tête de vis 2 uniquement par la zone distale 3 et par rotation sur le filetage 5 pour permettre ensuite le vissage de la tête de vis 2 sur le filetage 8 pour venir en butée contre la collerette 9. Une telle disposition empêche l'utilisateur de démonter facilement l'ensemble de la vis conforme à l'invention.

Avantageusement, les encoches 14 de la tête de vis 2 et les encoches 11 de la collerette 9 sont alignées lorsque la tête de vis est en butée contre la collerette 9. A cet effet, le premier filet du filetage 8 est repéré angulairement avec les encoches 11. De même, le premier filet du taraudage 12 est repéré angulairement avec les encoches 14 et par rapport au premier filet du filetage 8.

La tête de vis 2 conforme à l'invention comporte également, tel que cela est bien connu dans la technique antérieure, un filetage extérieur 13, de préférence sur toute sa longueur et dont le pas est plus faible que son pas interne 12. Le filetage externe 13 est donc également inférieur au pas des filetages 5 et 8, respectivement des zones distale 3 et proximale 7.

Selon l'invention, la zone distale 3 et la zone proximale 7 sont séparées par une zone centrale 15 conformée pour être inactive lors du vissage de la vis conforme à l'invention de manière à former une zone de glissement pour les fragments d'os à réunir et à mettre en compression.

Selon une version particulièrement avantageuse de l'invention, tel que montré aux figures 1 et 2, la zone centrale 15 est formée par une zone dépourvue de filetage, et de préférence par une zone sensiblement cylindrique et sensiblement lisse. Grâce à cette conformation, les deux fragments d'os à réunir et compresser peuvent donc librement se rapprocher relativement sans être soumis à une interface quelconque susceptible de limiter ou freiner leur mouvement.

Tel que cela est particulièrement visible aux figures 1 et 2, la zone centrale 15 se présente donc sous la forme d'une portion cylindrique lisse de diamètre inférieur au diamètre extérieur des filets 5A et 8A composant le filetage des zones distale 3 et proximale 7.

Selon une autre caractéristique particulièrement intéressante de l'invention, la zone centrale 15 s'étend sur une longueur variable dépendant de la longueur hors tout du corps de vis 1, et représentant de manière préférentielle entre 27 % et 60 % de la longueur totale du corps de vis 1. Tel que cela a été décrit précédemment, les filetages 5 et 8, respectivement de la zone distale 3 et de la zone proximale 7 sont de pas égaux. Néanmoins, selon une caractéristique intéressante de l'invention, les filetages 5 et 8 peuvent être avantageusement de profils différents.

Une telle variation des profils de filetage permet d'adapter précisément chaque filetage à sa fonction prioritaire, sans limiter le libre passage de la tête de vis 2 puisque les pas de filetages 5 et 8 demeurent égaux.

Ainsi, selon l'invention, il est particulièrement avantageux de réaliser le filetage 5 de la zone distale 3 avec une inclinaison plus importante des filets 5A le composant que le pas du filetage 8 de la zone proximale 7.

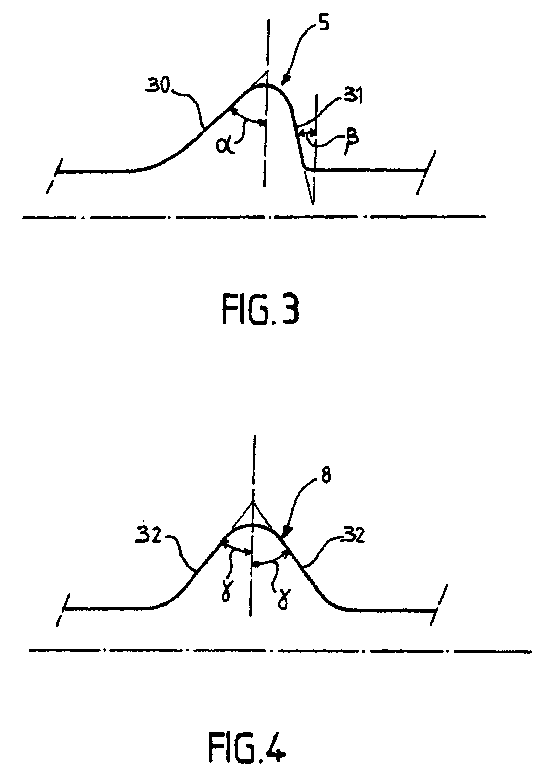

Avantageusement, le filetage de la zone distale 5 est un filetage osseux à pans inclinés (figure 3). A titre d'exemple préférentiel, l'inclinaison du pan avant 30 du filetage 5 sera de l'ordre de 30° (angle α) environ avec la verticale et de l'ordre de 3° (angle β) environ pour le pan arrière 31. Une telle réalisation permet d'obtenir une excellente tenue du corps de vis tout en minimisant l'évacuation éventuelle de débris osseux, ce qui réduit les phénomènes potentiels de résorption osseuse.

Avantageusement, le filetage 8 de la zone proximale 7 sera formée de filets isométriques dont les pans 32 forment par exemple un angle y de 30° environ chacun par rapport à la verticale. Grâce à cette réalisation, on obtient un déplacement particulièrement facile de la tête de vis 2 sur le filetage 8.

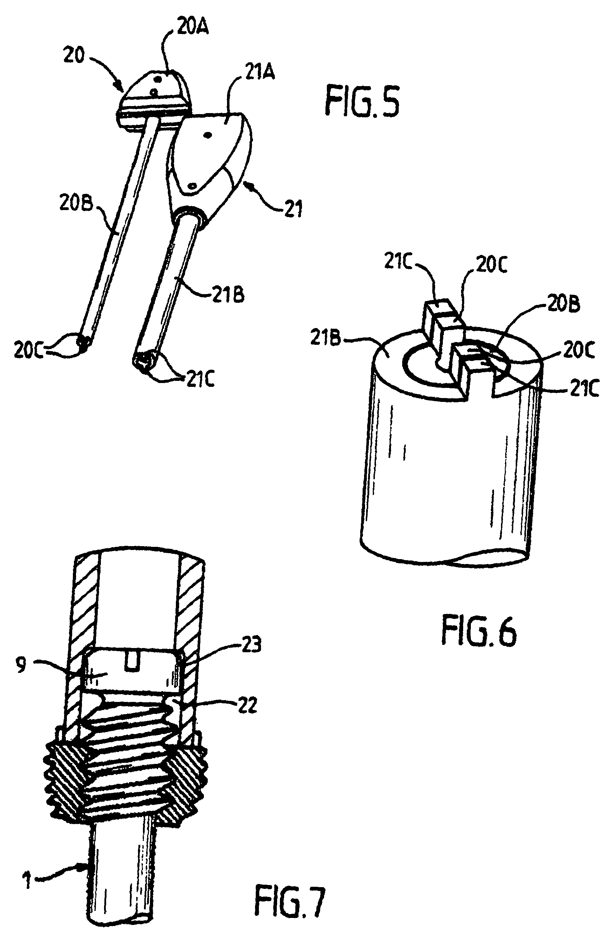

Tel qu'illustré notamment aux figures 5 à 7, l'invention se propose également de fournir un nouvel ensemble d'instruments assurant la mise en oeuvre de la vis d'ostéosynthèse conforme à l'invention. Selon l'invention, les instruments de mise en oeuvre de la vis d'ostéosynthèse comprennent en association, tel qu'illustré à la figure 5, un ensemble de deux tournevis 20, 21, formant respectivement un premier tournevis 20 destiné à assurer le vissage du corps de vis 1, et un second tournevis 21 destiné à assurer le vissage de la tête de vis 2. Chaque tournevis 20, 21 se compose d'une tête de manoeuvre, respectivement 20A, 21A, destinée à assurer la préhension manuelle du tournevis pour assurer le vissage. Chaque tête de manoeuvre 20A, 21A, est solidaire d'une tige 20B, 21B, creuse emmanchée dans chaque tête de manoeuvre 20A, 21A. L'extrémité travaillante de chaque tige 20B, 21B, est pourvue de deux ergots 20C, 21C, diamétralement opposés, de même hauteur et constituant les pièces travaillantes de chaque tournevis.

Le diamètre de la tige 20B du premier tournevis est inférieur au diamètre de la tige 21B du second tournevis 21 de manière à permettre d'emmancher le premier tournevis 20 axialement dans le second tournevis tel que cela est montré en particulier à la figure 6. Les têtes de manoeuvre 20A et 21A sont également conformées de manière à pouvoir être intégrées l'une dans l'autre pour ne former qu'une seule tête de manoeuvre en vue d'obtenir un seul instrument de vissage séparable selon l'utilisation souhaitée.

De manière avantageuse, lors de la mise en place, les ergots 20C, 21C des tiges 20B, 20C sont dans le prolongement les uns des autres (figure 6).

Selon l'invention, le second tournevis 21 est pourvu, tel qu'illustré à la figure 7, à sa partie terminale travaillante d'un lamage interne 22 destiné à recevoir la collerette 9 du corps de vis 1 en position de butée contre son fond 23, ledit lamage étant adapté sur le plan dimensionnel aux dimensions du corps de vis 1.

Ainsi, selon l'invention, le lamage interne 22 est d'une profondeur telle que lorsque la collerette 9 vient en butée contre le fond 23 lors du vissage de la tête de vis 2, le vissage de cette dernière est automatiquement bloqué avant d'avoir atteint le dernier filet du filetage 8 de manière à empêcher la sortie de la tête de vis 2 dudit filetage 8. Une telle particularité de conception est particulièrement utile puisqu'elle permet d'éviter, lors de la mise en place la désolidarisation de la vis de son filetage de vissage. Selon une version particulièrement avantageuse de l'invention, la hauteur interne du lamage 22 sera telle que le vissage de la tête de vis 2 est automatiquement interrompu sans possibilité de translation ultérieure de la tête de vis 2 lorsque cette dernière est en prise avec le filetage 8 par la totalité de son taraudage interne 12, les derniers filets inférieurs respectifs de la tête de vis 2 et du filetage 8 étant de préférence en prise. Une telle position est précisément illustrée à la figure 7 et permet de conserver une stabilité maximale à la tête de vis 2 puisque la totalité de son taraudage 12 est en prise avec le filetage 8.

Selon l'invention, lors de la mise en place, les deux tournevis 20, 21 tournent ensemble dans un premier temps (figure 6) pour visser le corps de vis 1 solidairement avec la tête de vis 2 jusqu'à l'enfouissement complet de cette dernière. Par la suite, le tournevis interne 20 est retiré afin que seul le tournevis externe 21 puisse visser la tête de vis 2 le long du corps de vis 1. Dans ce cas, le lamage interne 22 empêche la tête de vis 2 d'aller au-delà de la zone filetée, représentée par le filetage 8, la mise en butée de la collerette 9 contre le fond du lamage 23 arrêtant automatiquement la progression de la tête de vis 2.

Le fonctionnement de la vis d'ostéosynthèse à compression conforme à l'invention est le suivant.

Le corps de vis 1 et la tête de vis 2 étant livrés déjà montés, c'est-à-dire dans la configuration illustrée à la figure 2, le corps de vis 1 est introduit par vissage à l'aide d'un outil approprié dans le premier fragment d'os à percer. La rotation du corps de vis 1 permet à la zone distale 3 de traverser le premier fragment d'os puis également le second fragment osseux. Les filets 5A et 8A étant de pas identiques, les deux fragments osseux à réunir conservent dans cette première phase de pénétration une position relative inchangée. Lorsque la tête de vis 2 commence à pénétrer dans la première partie osseuse par l'intermédiaire de son filetage externe 13, les deux fragments d'os à réunir vont se rapprocher en raison du pas plus faible dudit filetage 13 comparativement au filetage 5 et 8. Il se produit en conséquence non seulement un rapprochement entre les deux fragments d'os, mais encore une mise en compression. Au cours de cette compression, la présence de la zone centrale 15 dépourvue de toute relation d'interface avec les deux fragments d'os qui se joignent autour d'elle par leur trait de fracture, permet une grande liberté de positionnement et de glissement des deux fragments osseux. En effet, au niveau de la zone centrale 15, aucune action parasite de frottement intervient, ce qui permet d'augmenter sensiblement la compression relative entre les deux fragments osseux à réunir et à ressouder.

Par la suite, une seconde mise en compression peut être effectuée par la mise en rotation, à l'aide d'un outil ancillaire spécifique, de la tête de vis 2 seule, c'est-à-dire indépendamment du corps de vis 1. Dans un tel cas, l'existence d'un filetage 8 isométrique permet un excellent contrôle, une bonne progression, et une bonne stabilisation de la tête de vis sur le corps de vis, gage d'une bonne maîtrise de la progression et du contrôle de la compression.

A titre d'exemple, la longueur de la zone distale 3 pourra être de l'ordre de 15 mm (avec un minimum de huit filets) pour assurer un bon ancrage osseux, la longueur hors tout de la tête de vis 2 étant d'environ 8 mm pour assurer une progression dans l'os de 6 mm, la longueur de la zone centrale 15 étant comprise entre 11 et 52 mm environ et la longueur de la zone proximale 7 étant de 14 mm environ.

## Revendications

1. Vis d'ostéosynthèse destinée à la mise sous compression de fragments d'os comprenant d'une part un corps de vis (1) présentant un filetage avec une zone distale (3) et une zone proximale (7), et d'autre part une tête de vis (2) destinée à être enfilée sur ledit corps de vis (1) et à coopérer avec la zone proximale (7) par un taraudage (12) interne congruent avec le filetage (8) de la zone proximale (7), **caractérisée en ce que** ladite tête de vis (2) comporte également un filetage externe (13) de pas inférieur à celui du corps de vis (1), la zone distale (3) et la zone proximale (7) étant séparées par une zone centrale (15) conformée pour être inactive lors du vissage de manière à former une zone de glissement pour les fragments d'os à mettre en compression, ladite tête de vis étant destinée à être enfilée sur ledit corps de vis (1) par la zone distale (3), ladite zone proximale (7) étant pourvue d'une butée pour la tête de vis (2).

2. Vis d'ostéosynthèse selon la revendication 1 **caractérisée en ce que** la zone centrale (15) est formée par une zone dépourvue de filetage.

3. Vis d'ostéosynthèse selon l'une des revendications 1 ou 2 **caractérisée en ce que** la zone centrale (15) est formée par une zone de surface sensiblement lisse.

4. Vis d'ostéosynthèse selon l'une des revendications 1 à 3 **caractérisée en ce que** la zone centrale (15) s'étend sur une longueur représentant entre 27 % et 60 % de la longueur totale du corps de vis (1).

5. Vis d'ostéosynthèse selon l'une des revendications 1 à 4 **caractérisée en ce que** les filetages (5, 8) respectivement de la zone distale (3) et proximale (7) sont de pas égaux mais de profils différents.

6. Vis d'ostéosynthèse selon la revendication 5 **caractérisée en ce que** le pas du filetage (5) de la zone distale (3) a une inclinaison plus importante que le pas du filetage (8) de la zone proximale (7).

7. Vis d'ostéosynthèse selon l'une des revendications 5 ou 6 **caractérisée en ce que** le filetage (5) de la zone distale (3) est un filetage osseux à pans inclinés.

8. Vis d'ostéosynthèse selon la revendication 7 **caractérisée en ce que** l'inclinaison des pans du filetage (5) par rapport à la verticale est de l'ordre de 30° environ pour le pan avant (30), et de l'ordre de 3° pour le pan arrière (31).

9. Vis d'ostéosynthèse selon l'une des revendications 1 à 8 **caractérisé en ce que** le taraudage (12) comporte un pas identique à celui du filetage (5) de la zone distale (3) afin de permettre à la tête de vis (2) d'être enfilée et déplacée par vissage sur ce dernier.

10. Vis d'ostéosynthèse selon l'une quelconque des revendications 1 à 9 **caractérisée en ce que** la butée est formée d'une collerette (9) contre laquelle la tête de vis (2) est destinée à venir à butée, la collerette (9) et la tête de vis (2) comportant respectivement deux encoches (11, 14) diamétralement opposées, lesdites encoches (11, 14) étant alignées lorsque la tête de vis (2) est en butée contre la collerette (9).

11. Vis d'ostéosynthèse selon l'une des revendications 5 à 10 **caractérisée en ce que** le filetage (8) de la zone proximale (7) est formé de filets isométriques, dont les pans (32) forment, par exemple, un angle de 30° environ par rapport à la verticale.

12. Ancillaire pour la mise en oeuvre de la vis d'ostéosynthèse selon l'une des revendications 1 à 11 comportant un premier tournevis (20) destiné à assurer le vissage du corps de vis et un second tournevis (21) destiné à assurer le vissage de la tête de vis, le premier tournevis (20) étant emmanché axialement dans le second tournevis pour permettre le vissage simultané du corps de vis (1) et de la tête de vis (2) et séparable de ce dernier pour le vissage individuel de la tête de vis (2), **caractérisé en ce que** le second tournevis (21) est pourvu à sa partie terminale travaillante d'un lamage interne (22) destiné à recevoir la butée du corps de vis (1) en position de butée contre son fond (23), ledit lamage étant d'une profondeur telle que lorsque la butée vient en butée contre le fond (23) lors du vissage de la tête de vis (2), le vissage de cette dernière est automatiquement bloqué avant d'avoir atteint le dernier filet du filetage (8) de manière à empêcher la sortie de la tête de vis (2) hors dudit filetage (8).

13. Ancillaire selon la revendication 12 **caractérisé en ce que** la profondeur du lamage (22) est telle que le vissage de la tête de vis (2) est automatiquement interrompu lorsque la totalité du taraudage (12) est en prise avec le filetage (8).

## Claims

1. Osteosynthesis screw intended for putting bone fragments under compression, comprising on the one hand a screw body (1) having a thread with a distal zone (3) and a proximal zone (7), and on the other hand a screw head (2) intended to be slipped onto the said screw body (1) and to cooperate with the proximal zone (7) through an internal tapping (12) congruent with the thread (8) on the proximal zone (7), **characterised in that** the said screw head (2) also comprises an external thread (13) with a thread smaller than that of the screw body (1), the distal zone (3) and the proximal zone (7) being separated by a central zone (15) conformed so as to be inactive during screwing so as to form a sliding zone for the bone fragments to be put under compression, the said screw head being intended to be slipped onto the said screw body (1) by means of the distal zone (3), the said proximal zone (7) being provided with a stop for the screw head (2).

2. Osteosynthesis screw according to Claim 1, **characterised in that** the central zone (15) is formed by a zone with no thread.

3. Osteosynthesis screw according to one of Claims 1 or 2, **characterised in that** the central zone (15) is formed by a zone with a substantially smooth surface.

4. Osteosynthesis screw according to one of Claims 1 to 3, **characterised in that** the central zone (15) extends over a length representing between 27% and 60% of the total length of the screw body (1).

5. Osteosynthesis screw according to one of Claims 1 to 4, **characterised in that** the threads (5, 8) respectively on the distal zone (3) and proximal zone (7) have equal pitches but different profiles.

6. Osteosynthesis screw according to Claim 5, **characterised in that** the pitch of the thread (5) on the distal zone (3) has a greater inclination than the pitch of the thread (8) on the proximal zone (7).

7. Osteosynthesis screw according to one of Claims 5 or 6, **characterised in that** the thread (5) on the distal zone (3) is a bone thread with inclined faces.

8. Osteosynthesis screw according to Claim 7, **characterised in that** the inclination of the faces of the thread (5) with respect to the vertical is around 30° approximately for the front face (30), and around 3° for the rear face (31).

9. Osteosynthesis screw according to one of Claims 1 to 8, **characterised in that** the thread (12) comprises a pitch identical to that of the thread (5) of the distal zone (3) in order to enable the screw head (2) to be slipped and moved by screwing onto the latter.

10. Osteosynthesis screw according to any one of Claims 1 to 9, **characterised in that** the stop is formed by a collar (9) against which the screw head (2) is intended to come to abut, the collar (9) and the screw head (2) comprising respectively two diametrically opposed notches (11, 14), the said notches (11, 14) being aligned when the screw head (2) is in abutment against the collar (9).

11. Osteosynthesis screw according to one of Claims 5 to 10, **characterised in that** the thread (8) on the proximal zone (7) is formed from isometric threads whose faces (32) form, for example, an angle of approximately 30° with respect to the vertical.

12. Ancillary device for using the osteosynthesis screw according to one of Claims 1 to 11, comprising a first screwdriver (20) intended to screw the screw body and a second screwdriver (21) intended to screw the screw head, the first screwdriver (20) being axially fitted in the second screwdriver in order to allow the simultaneous screwing of the screw body (1) and screw head (2) and separable from the latter for the individual screwing of the screw head (2), **characterised in that** the second screwdriver (21) is provided at its working end part with an internal countersink (22) intended to receive the stop on the screw body (1) in the position of abutment against its bottom (23), the said countersink having a depth such that, when the stop comes into abutment against the bottom (23) during the screwing of the screw head (2), the screwing of the latter is automatically locked before having reached the last thread on the thread (8) so as to prevent the emergence of the screw head (2) out of the said thread (8).

13. Ancillary device according to Claim 12, **characterised in that** the depth of the countersink (22) is such that the screwing of the screw head (2) is automatically interrupted when the entire tapping (12) is in engagement with the thread (8).

## Patentansprüche

1. Knochenschraube zur Kompression von Knochenteilen mit
- einerseits einem Schraubenkörper (1), der ein Gewinde mit einem Endbereich (3) und einem anliegenden Bereich (7) aufweist, und
- andererseits einem Schraubenkopf (2), der vorgesehen ist auf den Schraubenkörper (1) aufgesetzt zu werden und mit dem anliegenden Bereich (7) mittels einer inneren Verschraubung (12) zusammen zu wirken, die übereinstimmend ist mit dem Gewinde (8) des anliegenden Bereichs (7),
**dadurch gekennzeichnet, daß**
der Schraubenkopf (2) auch ein äußeres Gewinde (13) aufweist mit einer geringeren Steigung als der des Schraubenkörpers (1), wobei der Endbereich (3) und der anliegende Bereich (7) getrennt sind durch einen zentralen Bereich (15), der so ausgebildet ist, dass er inaktiv ist beim Schrauben derart, dass ein Gleitbereich für die zu komprimierenden Knochenteile gebildet ist, wobei der Schraubenkopf (2) vorgesehen ist, auf den Schraubenkörper (1) aufgesetzt zu werden über den Endbereich (3) und der anliegenden Bereich (7) mit einem Anschlag für den Schraubenkopf (2) versehen ist.

2. Knochenschraube gemäß Anspruch 1, **dadurch gekennzeichnet, dass** der zentrale Bereich (15) von einem Bereich gebildet ist, der kein Gewinde aufweist.

3. Knochenschraube gemäß einem der Ansprüche 1 oder 2, **dadurch gekennzeichnet, dass** der zentrale Bereich (15) von einem Bereich gebildet ist mit einer im wesentlichen glatten Oberfläche.

4. Knochenschraube gemäß einem der Ansprüche 1 bis 3, **dadurch gekennzeichnet, dass** der zentrale Bereich (15) sich über eine Länge erstreckt, die zwischen 27% und 60% der gesamten Länge des Schraubenkörpers ist.

5. Knochenschraube gemäß einem der Ansprüche 1 bis 4, **dadurch gekennzeichnet, dass** die Gewinde (7, 8) jeweils des Endbereichs (3) und des anliegenden Bereichs (7) gleiche Steigungen aber unterschiedliche Profile aufweisen.

6. Knochenschraube gemäß Anspruch 5, **dadurch gekennzeichnet, dass** die Gewindesteigung (5) des Endbereichs (3) eine größere Steigung hat als die Gewindesteigung (8) des anliegenden Bereichs (7).

7. Knochenschraube gemäß einem der Ansprüche 5 oder 6, **dadurch gekennzeichnet, dass** das Gewinde (5) des Endbereichs (3) ein Knochengewinde ist mit geneigten Flächen.

8. Knochenschraube gemäß Anspruch 7, **dadurch gekennzeichnet, dass** die Neigung der Flächen des Gewindes (5) mit Bezug zur Vertikalen ungefähr in der Größenordnung von 30° für die vordere Fläche (30) und in der Größenordnung von 3° für die hintere Fläche (31) ist.

9. Knochenschraube gemäß einem der Ansprüche 1 bis 8, **dadurch gekennzeichnet, dass** die Verschraubung (12) eine identische Steigung zu der des Gewindes (5) des Endbereichs (3) aufweist, um es dem Schraubenkopf (2) zu ermöglichen, mittels Schrauben auf den Letzteren aufgesetzt und verschoben zu werden.

10. Knochenschraube gemäß einem der Ansprüche 1 bis 9, **dadurch gekennzeichnet, dass** der Anschlag gebildet ist von einem Kragen (9), der dazu vorgesehen ist, dass der Schraubenkopf (2) daran zur Anlage kommt, wobei der Kragen (9) und der Schraubenkopf (2) jeweils zwei sich diametral gegenüber liegende Einschnitte (11, 14) aufweisen und diese Einschnitte (11, 14) ausgerichtet sind, wenn der Schraubenkopf (2) in Anlage ist an dem Kragen (9).

11. Knochenschraube gemäß einem der Ansprüche 5 bis 10, **dadurch gekennzeichnet, dass** das Gewinde (8) des anliegenden Bereichs (7) gebildet ist von isometrischen Gewinden, von denen die Flächen (32) zum Beispiel ungefähr einen Winkel von 30° mit Bezug zur Vertikalen bilden.

12. Hilfsinstrument zur Befestigung der Knochenschraube gemäß einem der Ansprüche 1 bis 11 mit einem ersten Schraubenzieher (20), der dazu vorgesehen ist die Verschraubung des Schraubenkörpers zu gewährleisten und einem zweiten Schraubenzieher (21), der dazu vorgesehen ist die Verschraubung des Schraubenkopfs zu gewährleisten, wobei der erste Schraubenzieher (20) axial in den zweiten Schraubenzieher eingesetzt ist, um das gleichzeitige Verschrauben des Schraubenkörpers (1) und des Schraubenkopfs (2) zu ermöglichen und trennbar von dem Letztern für das gesonderte Verschrauben des Schraubenkopfs (2), **dadurch gekennzeichnet, dass** der zweite Schraubenzieher (21) an seinem Endabschnitt zum Einsetzen mit einer inneren Ausnehmung (22) versehen ist, die dazu vorgesehen ist, den Anschlag des Schraubenkörpers (1) aufzunehmen in Anlageposition gegen seinen Boden (23), wobei die Ausnehmung eine solche Tiefe hat, dass wenn der Anschlag zur Anlage kommt gegen den Boden (23) beim Schrauben des Schraubenkopfes (2) das Gewinde des Letzteren automatisch blockiert ist bevor die letzte Windung des Gewindes (8) erreicht ist, so dass der Austritt des Schraubenkopfs (2) aus dem Gewinde (8) verhindert ist.

13. Hilfsinstrument gemäß Anspruch 12, **dadurch gekennzeichnet, dass** die Tiefe der Ausnehmung (22) so ist, dass das Schrauben des Schraubenkopfs (2) automatisch unterbrochen ist, wenn die Gesamtheit der Verschraubung (12) im Eingriff ist mit dem Gewinde (8).
